# EUROPÄISCHE PATENTSCHRIFT

(11) **EP 2 049 275 B1**
(45) Veröffentlichungstag und Bekanntmachung des Hinweises auf die Patenterteilung: **18.04.2012**
(21) Anmeldenummer: 07801484.2
(22) Anmeldetag: 01.08.2007
(51) Int. Cl.: B08B 9/20, B65B 55/02, B67C 3/00

(54) **VERFAHREN ZUR STERILISATION VON REINRÄUMEN FÜR DIE BEHANDLUNG UND/ODER DAS FÜLLEN UND VERSCHLIESSEN VON BEHÄLTERN**
PROCESS FOR STERILIZING CLEANROOMS FOR THE TREATMENT AND/OR THE FILLING AND CLOSURE OF VESSELS
PROCÉDÉ DE STÉRILISATION DE SALLES PROPRES POUR LE TRAITEMENT ET/OU LE REMPLISSAGE ET LA FERMETURE DE RÉCIPIENTS

(30) Priorität: 04.08.2006 DE 102006036475
(43) Veröffentlichungstag der Anmeldung: 22.04.2009
(73) Patentinhaber: KHS GmbH, 44143 Dortmund (DE)
(72) Erfinder: SANGI, Daryoush, 20251 Hamburg (DE); HEROLD, Thomas, 22926 Ahrensburg (DE)
(86) Internationale Anmeldenummer: PCT/EP2007/006805
(87) Internationale Veröffentlichungsnummer: WO 2008/014991

(56) Entgegenhaltungen:
- EP-A- 0 243 073
- DE-A1- 4 425 219
- JP-A- 8 091 490
- JP-A- 11 208 782
- US-A- 5 173 259

## Beschreibung

Die Erfindung betrifft ein Verfahren zur Sterilisation von Reinräumen für die Behandlung und/oder Füllung von Behältern.

Bei der Behandlung und Füllung von Behältern, beispielsweise mit Getränken oder Arzneimitteln, spielen aseptische Techniken eine zunehmende Rolle. Um eine lange Haltbarkeit des Behälterinhaltes bei gleichzeitigem Verzicht auf chemische Konservierungsstoffe oder sonstige Konservierungsverfahren, wie einer Hitzebehandlung oder ähnlichem, zu vermeiden, ist es entscheidend, dass sowohl die Behälter selber, als auch die Behandlungs-, Füll- oder sonstigen Anlegen, die mit dem Behälter in Berührung kommen, möglichst keimfrei sind. Dazu werden die Behandlungs- oder Füllanlagen eingehaust und unter Überdruck gesetzt. Außerdem wird dafür gesorgt, dass nur keimfreie Stoffe ins Innere gelangen können, beispielsweise durch die Verwendung von Luftfiltern, Schleusen und dgl.

Zur Gewährleistung einer möglichst keimfreien Umgebung muss das Innere derartiger Anlagen zudem regelmäßig sterilisiert werden. Dabei kommen unterschiedliche Verfahren zum Einsatz. Üblicherweise wird ein chemisches Sterilisationsmedium über im Inneren angebrachte Sprühköpfe während eines Reinigungszyklusses ins Innere der Anlage eingebracht, wo es mit eventuell vorhandenen unerwünschten Keimen und dgl. reagiert und diese abtötet. Um eine möglichst vollständige Reinigung des Innenraumes zu ermöglichen, sind dabei eine Vielzahl von Sprühköpfen nötig, damit alle Bereiche der zum Teil kompliziert aufgebauten Vorrichtungen im Inneren der Anlage wirksam sterilisiert werden können. Als Sterilisationsmedium kommt beispielsweise Per-Essigsäure zum Einsatz.

Nachteilig an dieser Art der Sterilisation ist, dass eine Vielzahl von Sprühköpfen im Inneren der Maschine angeordnet sein muss, um eine vollständige Benetzung aller Flächen mit dem Desinfektionsmittel zu ermöglichen. Zudem sind die teilweise verwendeten flüssigen Sterilisationsmedien in ihrer Wirkung oft nicht ausreichend oder nicht zur Verwendung im Lebensmittelbereich freigegeben.

Ein Verfahren mit den Merkmalen des Oberbegriffs des Patentanspruches 1 ist bekannt (EP 0243 073 A1). Bei diesern bekannten Verfahren wird das zum Sterilisieren von Behältern in Form von Kartons verwendete, H2O2 enthaltende Sterilisationsmedium zugleich für die Sterilisation des Inneren des Reinraums der zum Sterilisieren, Füllen und Verschließen der Behälter ausgebildeten Anlage verwendet. Durch Umschalten eines Umschaltventils sowie durch Ansteuerung zahlreicher Drosseln in einem das Sterilisationsmedium sowie heiße Luft führenden Leitungssystem wird beim Sterilisieren des Reinraums das Sterilisationsmedium in den Raum zum Füllen der Behälter eingeleitet und strömt von dort in die Aktivierungs- und Sterilisationseinrichtung. Allein schon durch das umschaltbare Ventil und die Vielzahl von steuerbaren Drosseln erfordert das bekannte Verfahren einen erheblichen konstruktiven Aufwand.

Aufgabe der Erfindung ist es, ein Sterilisationsverfahren zur Verfügung zu stellen, das eine wirksame Sterilisation des Inneren der Reinräume ermöglicht, ohne dass aufwändige zusätzliche Reinigungsanlagen installiert werden müssen.

Die Erfindung erreicht dies durch ein Verfahren zur Sterilisation von Reinräumen für die Behandlung und/oder Füllung von Behältern mit den Merkmalen des Patentanspruches 1.

Im Inneren von Reinräumen für derartige erfindungsgemäße Anlagen befindet sich auch eine Einrichtung zur Sterilisation der zu behandelnden bzw. zu füllenden Behälter unter Verwendung eines dampfförmigen Sterilisationsmediums. Das Sterilisationsmedium wird dabei mindestens in das Innere und den oberen Randbereich des Behälters geleitet, wo es sich niederschlägt und einen Kondensationsfilm aufgrund der im Vergleich zum Sterilisationsmedium kühleren Behälteroberfläche bildet. In der Folge wird dem Sterilisationsmedium eine gewisse Wärmemenge T zugeführt, die das Medium aktivieren kann, so dass in der Folge die eigentliche Sterilisationsreaktion abläuft. Wenn als Sterilisationsmedium H₂O₂ verwendet wird, wird beispielsweise durch Wärmezufuhr eine Zerfallsreaktion gestartet, bei der das H₂O₂ zerfällt und die Zerfallszwischenprodukte freie Radikale beinhalten, die mit vorhandenen Keimen und sonstigen Verunreinigungen reagieren. Am Ende der Reaktion bleiben im Wesentlichen Wasser und möglicherweise einige Zerfallsprodukte zurück, wobei insbesondere keine schädlichen Keime und dgl. mehr vorhanden sind, so dass ein den Anforderungen für Lebensmittel oder Pharmazieanwendungen genügender Grad der Sterilität erreicht wird.

Nach der Erfindung werden die Anlagen zur Einleitung des dampfförmigen Sterilisationsmediums in die Behälter auch zum Sterilisieren der gesamten Anlage verwendet. Dazu wird das dampfförmige Sterilisationsmedium, beispielsweise H₂O₂, durch die Sterilisationseinrichtung im Leerbetrieb ohne Behälter an die Umgebung abgegeben, die sich daraufhin mit dem Medium anreichert. Genau wie in den Behältern schlägt sich das Medium auf allen Flächen, mit denen es in Berührung kommt, aufgrund der Temperaturdifferenz nieder und bildet dort einen Kondensationsfilm. Durch geeignete Luftführung wird dabei das austretende dampfförmige Medium durch die gesamte Anlage geleitet. Je nach Konfiguration der Anlage können dabei unterschiedliche Reihenfolgen zum Einsatz kommen. So kann durch vorhandene Türen zwischen einzelnen Bereichen der Anlage gesteuert werden, wohin und in welcher Menge das dampfförmige Medium transportiert werden soll. Dies wird unterstützt durch eine gesteuerte Zufuhr von gereinigter Frischluft, die das dampfförmige Medium mit sich in die unterschiedlichen Bereiche der Anlage führt. Analog kann durch vorhandene Absaugeinrichtungen ein Luftstrom im Inneren der Anlage erzeugt werden, der das dampfförmige Sterilisationsmedium mit sich in unterschiedliche Bereiche der Anlage leitet, wo es, wie beschrieben, einen Kondensationsfilm bilden kann.

Je nach Anlage kann es sinnvoll sein, die gesamte Anlage gleichmäßig mit einer ausreichenden Konzentration des dampfförmigen Sterilisationsmediums zu füllen oder unterschiedliche Bereiche der Anlage nacheinander zu versorgen.

Sobald eine ausreichende Menge des Sterilisationsmediums im Inneren der Anlage verteilt ist, kann dieses im Fall der Verwendung von H₂O₂ durch Zufuhr von Wärme aktiviert werden. Dies kann durch Zuführung von heißem Dampf oder heißer Luft geschehen. Dazu können an der Anlage Luftzuführ- und Filtereinrichtungen mit integrierter Heizeinrichtung vorgesehen sein, die entsprechende Mengen an heißer, gefilterter Luft ins Innere des Reinraumes bringen und so die Zerfallsreaktion des Sterilisationsmediums starten. Auch hier kann durch gleichzeitige Zuführung ausreichender Mengen von heißer Luft in alle Bereiche der Anlage die Reaktion im Wesentlichen überall gleichzeitig gestartet werden oder alternativ wieder eine bereichsweise Aktivierung in Schritten nacheinander erfolgen.

Nachdem die Reaktion beendet ist, können mittels vorhandener Absaugeinrichtungen die verbliebenen Reaktionsprodukte aus dem Inneren der Anlage abgesaugt werden. Auch hier wird wieder durch gezielte Luftführung dafür gesorgt, dass eine möglichst vollständige Entfernung aller Zerfallsprodukte und ggf. ein Austausch der Innenluft vorgenommen wird.

Je nach Ausgestaltung der Anlage können vorhandene Rohrleitungen ähnlich sterilisiert werden. Hier wird das dampfförmige Sterilisationsmedium durch die vorhandenen Rohrleitungen gepumpt und durch heiße Luft oder heißen Dampf aktiviert. Alternativ kann auch unmittelbar eine Mischung aus dem dampfförmigen Sterilisationsmedium und heißen Dampf durch die Rohrleitungen geleitet werden. Dieser Schritt kann vor der beschriebenen Sterilisation des Innenraumes durchgeführt werden, da dann eventuell aus den Rohrleitungen austretende Zerfallsprodukte mit den restlichen Zerfallsprodukten aus der Anlage entfernt werden.

Je nach Anlage ist es nötig, dass im Anschluss an die Behältersterilisationseinrichtung Aktivierungseinrichtungen vorgesehen sind, die den in die Behälter eingebrachten Kondensationsfilm durch Zuführung beispielsweise von heißem Dampf oder heißer Luft im Normalbetrieb aktivieren. Diese können im Sterilisationsbetrieb in Ausgestaltung ebenfalls dazu genutzt werden, das dampfförmige Sterilisationsmedium ins Innere der Anlage einzubringen, was den Vorgang erheblich beschleunigen kann.

Diese und weitere Ausgestaltungen der Erfindung ergeben sich aus den Unteransprüchen, die, genau wie der Hauptanspruch, zum Gegenstand der Beschreibung gemacht werden.

Die Erfindung betrifft zudem noch eine Reinigungseinrichtung zur Sterilisation von Reinräumen für die Behandlung und/oder Füllung von Behältern mit dem beschriebenen Verfahren sowie eine Steuerungseinrichtung zur Durchführung des beschriebenen Verfahrens. Durch eine derartige Reinigungseinrichtung und eine derartige Steuerungseinrichtung wird die gestellte Aufgabe ebenfalls in vorteilhafter Weise gelöst.

Weitere Vorteile und Ausgestaltungen der Erfindung ergeben sich aus der Zeichnung. Diese zeigt in
- Fig. 1: eine schematische Draufsicht auf eine Reinraumfüllanlage zur Verwendung mit dem erfindungsgemäßen Verfahren sowie in
- Fig. 2: ein schematisches Anschlussdiagramm der Anlage nach Fig. 1.

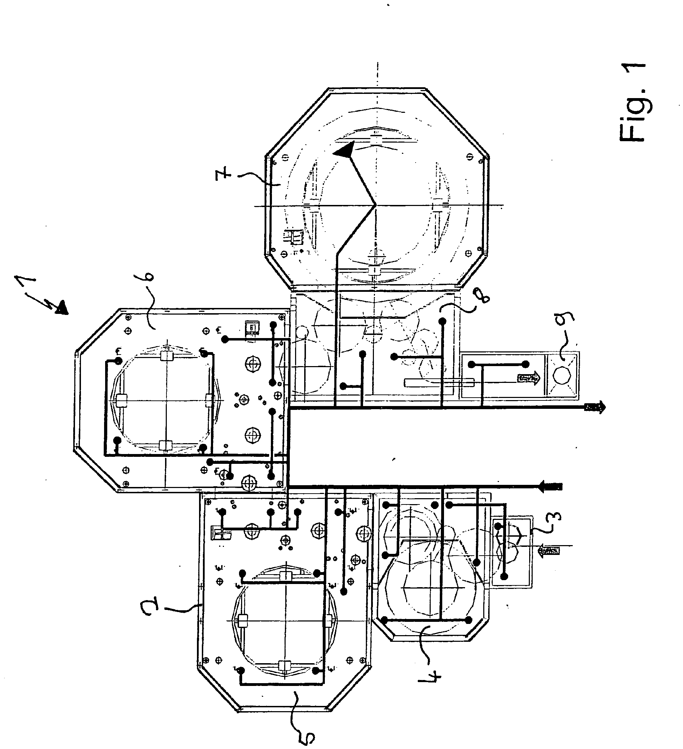

Eine allgemein mit 1 bezeichnete Anlage für das Befüllen von Behältern, insbesondere Flaschen oder Dosen mit Getränken und dgl., ist in,Fig. 1 näher dargestellt. Die gesamte Anlage ist mit einer Einhausung 2 umgeben, die den gesamten Innenraum vor Verschmutzungen von außen bewahrt, so dass im Inneren Reinraumbedingungen herrschen.

Die Anlage weist einen Behältereinlass 3 auf, durch den die zu befüllenden Flaschen ins Innere der Anlage gelangen können. Anschließend werden die Behälter in einer Sterilisationseinrichtung 4 sterilisiert. Sie laufen dazu in einer Kreisbahn um, zusammen mit einer Vielzahl von Verdampferköpfen, die dampfförmiges H₂O₂ ins Innere und den oberen Außenbereich des Behälters leiten. Aufgrund der Temperaturunterschiede zwischen dem verdampften H₂O₂ und der Behälterwand schlägt sich das Sterilisationsmittel nieder und bildet einen Kondensationsfilm. Im Anschluss werden die Behälter in eine erste Aktivierungseinrichtung 5 gefördert, in der heiße Luft oder heißer Dampf außen oder innen an den Behälter geleitet wird. Daraufhin wird eine Zerfallsreaktion in dem eingebrachten H₂O₂ gestartet, in der es über mehrere Zwischenstadien zerfällt. Während der Reaktion und durch die zwischenzeitlich gebildeten Reaktionsprodukte werden eventuell vorhandene Keime oder Verunreinigungen angegriffen und zersetzt, bis am Ende im Wesentlichen Wasser mit wenigen Zerfallsresten übrig bleibt.

Da die Reaktion eine gewisse Zeit in Anspruch nimmt, ist, nachdem die Behälter in einer Teilkreisbahn durch die erste Aktivierungseinrichtung 5 gelaufen sind im Anschluss eine zweite Aktivierungseinrichtung 6 angeordnet, in der die Behälter eine weitere Kreisbahn zurücklegen, so dass genügend Zeit vorhanden ist, damit die Zerfallsreaktion am Ende der zweiten Aktivierungseinrichtung 6 vollständig abgeschlossen ist. Im Anschluss daran laufen die Flaschen in die Fülleinrichtung 7, in der die Flaschen umlaufend mit dem Füllgut befüllt werden und im Anschluss daran in der Verschlusseinrichtung 8 verschlossen werden.

Danach verlassen sie die Füllanlage durch den Behälterauslass 9.

Um die Reinraumbedingungen im Inneren der Anlage 1 aufrechtzuerhalten, muss diese regelmäßig sterilisiert werden. Hierzu wird das erfindungsgemäße Verfahren wie folgt durchgeführt:

Zunächst wird der Produktionsbetrieb der Anlage gestoppt, also insbesondere die Zufuhr von Behältern unterbrochen und die unterschiedlichen Einrichtungen im Inneren der Anlage 1 heruntergefahren. Im Anschluss wird der Reinigungszyklus gestartet.

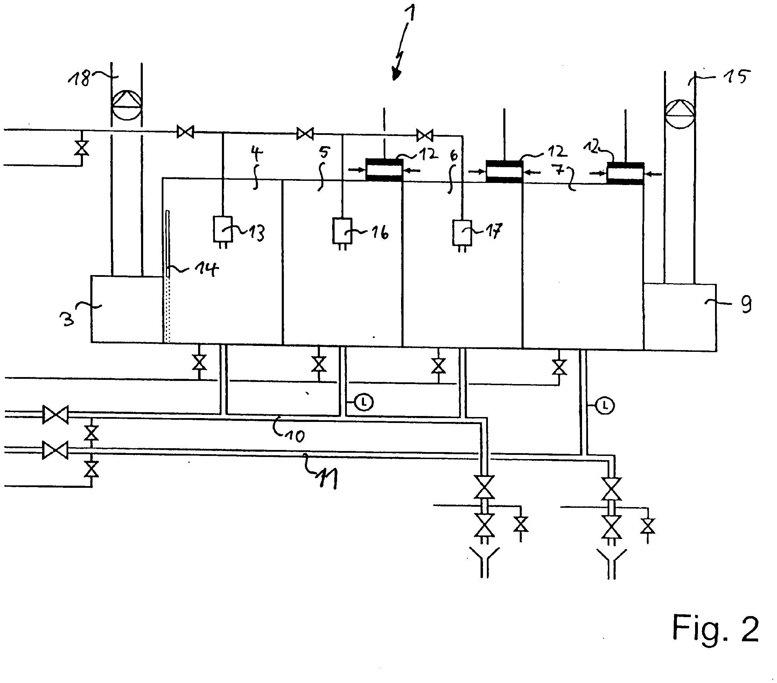

Wie in der schematischen Übersicht in Fig. 2 erkennbar, sind zur Versorgung des Inneren der Anlage 1 mit steriler Frischluft Filterbelüftungseinheiten 12 vorgesehen, die beispielsweise in Form von HEPA-Filtern ausgebildet sind.

In einer besonders vorteilhaften Ausgestaltung der vorliegenden Erfindung ist vorgesehen, dass auch diese Filter während eines Reinigungszyklus gereinigt werden. Dazu sind diese Filter mit einer Einrichtung ausgestattet, die die enthaltenen Filterschichten mit flüssigem oder dampfförmigem H₂O₂ versorgt. In einer besonderen Ausgestaltung weisen die Filterbelüftungseinheiten 12 eine integrierte Heizeinrichtung auf, die in der Folge aktiviert werden kann und so dass das H₂O₂ im Inneren des Filters aktiviert werden kann, was zur Folge hat, dass oben die beschriebene Sterilisationsreaktion auch im Inneren der Filterbelüftungseinheiten 12 ablaufen kann.

In einem zweiten Schritt wird dann die Sterilisationseinrichtung 4 der Anlage 1 wieder aktiviert. Da, wie ausgeführt, während der Sterilisation der Anlage keine Behälter durch das Innere der Anlage laufen, tritt dann das zur Sterilisation erzeugte dampfförmige H₂O₂ aus Sterilisationsköpfen 13 aus, und zwar direkt und ungehindert in das Innere des Raumes, der die Einrichtung 4 umgibt. Dort schlägt sich, wie beschrieben, das dampfförmige H₂O₂ an sämtlichen Flächen nieder und bildet dort einen Kondensationsfilm. Damit das Medium bzw. das dampfförmige H₂O₂ nicht nach außen gelangen kann, ist eine Verschlusstür 14 vorgesehen, die zu Beginn des Reinigungszyklus geschlossen wird.

Um das, aus den Sterilisationsköpfen 13 austretende, dampfförmige H₂O₂ in der gesamten Anlage 1 zu verteilen, werden zwischen den einzelnen Anlagenteilen ggf. vorhandene Trenntore gesteuert geöffnet und geschlossen. Um für einen gesteuerten Luftstrom im Inneren der Anlage zu sorgen, kann weiterhin eine beispielsweise am Auslass angeordnete Absaugeinrichtung 15 gesteuert aktiviert werden, so dass ein Luftstrom durch die Anlage 1 einsetzt, der das aus den Sterilisationsköpfen 13 austretende dampfförmige H₂O₂ durch die gesamte Anlage 1 transportiert, wo es, wie beschrieben, überall auf den Flächen kondensiert und einen Kondensationsfilm bildet.

In Ausgestaltung können zusätzlich die beiden Aktivierungseinrichtungen 5 und 6 zur Verteilung des dampfförmigen Wasserstoffperoxids (H₂O₂) genutzt werden. Da hier zur Aktivierung der Reaktion in den Behältern ebenfalls Abgabeköpfe 16 bzw. 17 vorgesehen sind, kann statt der im Normalbetrieb hierdurch abgegebenen heißen Luft oder dem heißen Dampf im Sterilisationszyklus dampfförmiges H₂O₂ abgegeben werden, so dass eine schnellere Befüllung des Anlageninnenraumes mit dem Sterilisationsmedium erreicht wird.

Danach werden die vorhandenen Leitungen 10, 11, beispielsweise für das Füllgut, mit einem Gemisch aus H₂O₂ und heißem Dampf beaufschlagt, so dass diese durch die oben beschriebene Sterilisationsreaktion sterilisiert werden. Zerfallsprodukte, wie Wasser und sonstige Reste, gelangen dabei durch die Rohrleitungen teilweise ins Innere der Anlage. Eine Steuerung sorgt dafür, dass bei Verzweigungen, Ventilen und dgl. alle Leitungsteile nacheinander mit dem Sterilisationsgemisch ausreichend versorgt werden.

Nachdem durch die gezielte Luftführung und der Abgabe einer entsprechenden Menge des Sterilisationsmediums eine ausreichende Konzentration im Inneren der Anlage erreicht worden ist, und sich demzufolge ein ausreichender Kondensationsfilm auf den Oberflächen in der Anlage gebildet hat, muss die Reaktion durch Zufuhr einer ausreichenden Wärmemenge aktiviert werden.

Hierzu werden die beschriebenen Filterbelüftungseinheiten 12 verwendet. Diese haben, wie beschrieben, in Ausgestaltung eine eingebaute Heizeinrichtung, und können so eine entsprechende Menge heißer gefilterter Luft ins Innere der Anlage leiten, bis die Reaktion anläuft. Alternativ kann auch eine externe Heizquelle vor die Filter geschaltet sein, die für die Sterilisation ausreichend heiße Luft durch die Filterbelüftungseinheiten leitet.

Nach dem Einbringen der entsprechenden Menge an heißer Luft beginnt die beschriebene Zerfallsreaktion des niedergeschlagenen und ggf. noch in der Anlage befindlichen dampfförmigen H₂O₂, das zu Wasser und Zerfallsresten zerfällt und während der Reaktion vorhandene Keime und sonstige Verunreinigungen zersetzt und somit die gesamte Anlage sterilisiert.

Nachdem die Reaktion abgeschlossen ist, werden etwaige vorhandene Reste des dampf- oder gasförmigen Sterilisationsmediums aus dem Inneren der Anlage entfernt, wobei es von besonderem Vorteil ist, die Anlage gleichzeitig zu trocknen.

Dazu ist vorgesehen, die vorhandenen Absaugeinrichtungen 15 und 18 zu aktivieren, sowie die Einlasstür 14 zu öffnen und über die beheizbaren Filterbelüftungseinheiten 12 heiße Luft in das Innere der Anlage einzubringen und durch die Absaugeinrichtungen 15, 18 wieder abzusaugen.

Dabei ist es von besonderem Vorteil, die heiße Luft der Anlage in ihrem Inneren, vorzugsweise etwas in der Mitte des Prozessweges zuzuführen, und anschließend durch die Absaugeinrichtungen 15 und 18 in Richtung der beiden Enden des Prozessweges, also in Richtung Einlauf 14 und in Richtung des Behälterauslasses 9 abzusaugen, so dass das Eindringen von Keimen durch diese Vorgehensweise zusätzlich erschwert wird.

Im Anschluss ist die Anlage sterilisiert, so dass sie wieder für den Produktionsbetrieb hochgefahren werden kann.

Die Erfindung ist nicht auf das vorstehende Ausführungsbeispiel beschränkt, sondern kann in vielfältiger Hinsicht abgewandelt werden, ohne den Grundgedanken zu verlassen. So ist beispielsweise die Konfiguration der Anlage in weiten Bereichen variabel. Auch die Ablauffolge kann verändert werden oder ein Teil der Schritte gleichzeitig ablaufen. Auch das verwendete Sterilisationsmedium kann abgewandelt werden, so lange es den Anforderungen an die Sterilisationsleistung und den Zweck der Anlage genügt.

So wurde vorstehend ausschließlich von einem dampfförmigen Sterilisationsmedium gesprochen, doch versteht es sich für den Fachmann von selbst, dass sich dass zuvor gesagte auch auf die Anwendung eines Sterilisationsmediums erstreckt, welches aerosolförmig ist. Ebenfalls kann sich das Sterilisationsmedium in einem Zustand befinden, in welchem die Aggregatzustände dampfförmig und flüssig gemeinsam oder gleichzeitig oder in ständigem Wechsel auftreten.

Wesentlich für den Aggregatzustand des Sterilisationsmediums ist lediglich, dass es sich nach dem Austritt aus seiner Quelle, beispielsweise den Sterilisationsköpfen 13, in das Innere des Reinraums dessen Innenwandungen mittels eines Kondensation- oder Niederschlagfilms möglichst vollständig benetzt.

### Bezugszeichenliste

- 1: Anlage
- 2: Einhausung
- 3: Behältereinlass
- 4: Sterilisationseinrichtung
- 5: erste Aktivierungseinrichtung
- 6: zweite Aktivierungseinrichtung
- 7: Fülleinrichtung
- 8: Verschlusseinrichtung
- 9: Behälterauslass
- 10: Leitung
- 11: Leitung
- 12: Filterbelüftungseinheit
- 13: Sterilisationskopf
- 14: Verschlusstür
- 15: Absaugeinrichtung
- 16: Abgabekopf
- 17: Abgabekopf
- 18: Absaugeinrichtung

## Patentansprüche

1. Verfahren zur Sterilisation von Reinräumen für das Sterilisieren und Füllen von Behältern, wobei im Inneren des Jeweiligen Reinraums zur Sterilisation der Behältern mittels eines dampf- oder aerosolförmigen Sterilisationsmediums wenigstens eine Sterilisationseinrichtung (4) mit Mitteln zur Einleitung des Sterilisationsmediums in die Behälter, eine Aktivierungseinrichtung (5) zum Aktivieren des Sterilisationsmediums und eine Fülleinrichtung (7) zum Füllen der Behälter gebildet sind, wobei zur Sterilisation des Reinraumes das für die Sterilisation der Behälter verwendete Sterilisationsmedium in den Reinraum abgegeben und durch den Reinraum so geleltet wird, dass Innerhalb des Reinraumes ein Nlederschlags- oder Kondensationsfilm mit dem Sterilisationsmedium gebildet wird,
**dadurch gekennzeichnet,**
**dass** beim Sterilisieren des Reinraums das Sterilisationsmedium über die Mitteln (13) zur Einleitung des Sterilisationsmediums in die Behälter (4) in die Sterilisationseinrichtung (4) eingebracht und aus dieser durch eine gezielte Luftführung In die Fülleinrichtung (7) geleitet wird.

2. Verfahren nach Anspruch 1, **dadurch gekennzeichnet, dass** zur gezielten Luftführung steuerbare Belüftungseinheiten (12) und/oder steuerbare Lufteinlässe und/oder steuerbare Luftauslässe und/oder steuerbare Türen (14) verwendet werden.

3. Verfahren nach Anspruch 1 oder 2, **dadurch gekennzeichnet, dass** das Sterilisationsmedium und/oder der Niederschlags- oder Kondensationsfilm durch Zufuhr eines Aktivierungsmediums aktiviert und die Sterilisationsreaktion gestartet wird.

4. Verfahren nach einem der vorangegangenen Ansprüche, **dadurch gekennzeichnet, dass** als Sterilisationsmedium dempfförmiges H₂O₂ verwendet wird.

5. Verfahren nach einem der vorangegangenen Ansprüche, **dadurch gekennzeichnet, dass** als Aktivierungsmedium erhitzte Luft und/oder Gas und/ oder Dampf verwendet wird.

6. Verfahren nach einem der vorangegangenen Ansprüche, **dadurch gekennzeichnet, dass** vorhandene Rohrleitungen (10,11) mittels desselben Sterilisations- und/oder Aktivierungsmediums sterilisiert werden.

7. Verfahren nach einem der vorangegangenen Ansprüche,
**dadurch gekennzeichnet, dass** in dem Reinraum wenigstens eine Verschließeinrichtung (8) verwendet wird.

8. Verfahren nach einem der vorangegangenen Ansprüche, **dadurch gekennzeichnet, dass** das dampfförmigen Sterilisationsmedium zusätzlich aus der Aktivierungseinrichtung (5) in die Umgebung abgegeben wird.

9. Verfahren nach einem der vorangegangenen Ansprüche, **dadurch gekennzeichnet, dass** im Reinraum befindliche Reste des Sterilisationsmediums und/oder Zerfallsprodukte mittels wenigstens einer Absaugeinrichtung (15,18) abgesaugt werden.

10. Verfahren nach einem der vorangegangenen Ansprüche, **dadurch gekennzeichnet, dass** zur Absaugung im Reinraum befindlicher Reste des Sterillsationsmediums und/oder Zerfallsprodukte und/oder zur Trocknung des Reinraumes auf der Behandlungsstrecke, vorzugsweise in deren Mitte, erhitzte Luft oder erhitztes Gas zugeführt wird, weiches in Richtung eines Behältereinlasses (3) und/oder in Richtung eines Behälterauslasses (9) abgesaugt wird.

## Claims

1. Process for sterilizing cleanrooms for the sterilization and filling of containers, wherein formed in the interior of the respective cleanroom for sterilizing the containers by means of a sterilization medium in vapor or aerosol form are at least one sterilizing device (4) having means for introducing the sterilization medium into the container, an activation device (5) for activating the sterilization medium, and a filling device (7) for filling the containers, wherein in order to sterilize the cleanroom the sterilization medium used for sterilizing the containers is discharged into the cleanroom and is conducted through the cleanroom in such a way that a precipitation or condensation film comprising the sterilization medium is formed within the cleanroom, **characterized in that**, during the sterilization of the cleanroom, the sterilization medium is fed into the sterilizing device (4) via the means (13) for introducing the sterilization medium into the containers (4) and is conducted from said sterilizing device into the filling device (7) through targeted air guidance.

2. Process according to claim 1, **characterized in that**, for the targeted air guidance, use is made of controllable ventilation units (12) and/or controllable air inlets and/or controllable air outlets and/or controllable doors (14).

3. Process according to claim 1 or 2, **characterized in that** the sterilization medium and/or the precipitation or condensation film is activated by supplying an activation medium and the sterilization reaction is started.

4. Process according to any one of the preceding claims, **characterized in that** H₂O₂ in vapor form is used as the sterilization medium.

5. Process according to any one of the preceding claims, **characterized in that** heated air and/or gas and/or vapor is used as the activation medium.

6. Process according to any one of the preceding claims, **characterized in that** pipelines (10, 11) which are present are sterilized by means of the same sterilization and/or activation medium.

7. Process according to any one of the preceding claims, **characterized in that** at least one closing device (8) is used in the cleanroom.

8. Process according to any one of the preceding claims, **characterized in that** the sterilization medium in vapor form is additionally discharged from the activation device (5) into the environment.

9. Process according to any one of the preceding claims, **characterized in that** residues of the sterilization medium and/or decomposition products located in the cleanroom are sucked off by means of at least one suction device (15, 18).

10. Process according to any one of the preceding claims, **characterized in that**, in order to suck off residues of the sterilization medium and/or decomposition products located in the cleanroom and/or in order to dry the cleanroom, heated air or heated gas is fed along the treatment path, preferably in the center thereof, and is sucked off in the direction of a container inlet (3) and/or in the direction of a container outlet (9).

## Revendications

1. Procédé de stérilisation de salles propres pour la stérilisation et le remplissage de conteneurs, dans lequel sont formés à l'intérieur de la salle propre respective, pour la stérilisation des conteneurs au moyen d'un milieu de stérilisation à la vapeur ou sous forme d'aérosol, au moins un dispositif de stérilisation (4) comportant des moyens d'acheminement du milieu de stérilisation dans les conteneurs, un dispositif d'activation (5) pour activer le milieu de stérilisation et un dispositif de remplissage (7) pour remplir les conteneurs, dans lequel, pour la stérilisation de la salle propre, le milieu de stérilisation utilisé pour la stérilisation des conteneurs est déposé dans la salle propre et acheminé dans la salle propre de telle sorte que dans la salle propre, un film de précipitation ou de condensation soit formé avec le milieu de stérilisation,
**caractérisé en ce que**
lors de la stérilisation de la salle propre, le milieu de stérilisation est inséré par les moyens (13) d'acheminement du milieu de stérilisation dans les conteneurs (4) dans le dispositif de stérilisation (4) et est conduit de celui-ci par une canalisation d'air ciblée dans le dispositif de remplissage (7).

2. Procédé selon la revendication 1, **caractérisé en ce que**, pour la canalisation d'air ciblée, des unités d'aération commandables (12) et/ou des entrées d'air commandables et/ou des sorties d'air commandables et/ou des portes commandables (14) sont utilisées.

3. Procédé selon la revendication 1 ou 2, **caractérisé en ce que** le milieu de stérilisation et/ou le film de précipité ou de condensation est activé par l'amenée d'un milieu d'activation et la réaction de stérilisation est initiée.

4. Procédé selon l'une des revendications précédentes, **caractérisé en ce que** l'on utilise comme milieu de stérilisation, du H₂O₂ sous forme de vapeur.

5. Procédé selon l'une des revendications précédentes, **caractérisée en ce que** l'on utilise comme milieu d'activation, de l'air chauffé et/ou du gaz et/ou de la vapeur.

6. Procédé selon l'une des revendications précédentes, **caractérisé en ce que** les conduites présentes (10, 11) sont stérilisées au moyen du même milieu de stérilisation et/ou d'activation.

7. Procédé selon l'une des revendications précédentes, **caractérisé en ce que**, dans la salle propre, on utilise au moins un dispositif de fermeture (8).

8. Procédé selon l'une des revendications précédentes, **caractérisé en ce que** le milieu de stérilisation sous forme de vapeur est en outre déposé du dispositif d'activation (5) dans l'environnement.

9. Procédé selon l'une des revendications précédentes, **caractérisé en ce que** les résidus de milieu de stérilisation et/ou des produits de décomposition se trouvant dans la salle propre sont aspirés par au moins un dispositif d'aspiration (15, 18).

10. Procédé selon l'une des revendications précédentes, **caractérisé en ce que**, pour l'aspiration des résidus de milieu de stérilisation et/ou des produits de décomposition se trouvant dans la salle propre et/ou pour le séchage de la salle propre sur le parcours de traitement, de préférence en son milieu, de l'air chaud ou du gaz chauffé qui est aspiré dans le sens d'une entrée du conteneur (3) et/ou dans le sens d'une sortie du conteneur (9), est acheminé.
